# EUROPÄISCHE PATENTANMELDUNG

(11) **EP 0 856 281 A2**
(43) Veröffentlichungstag der Anmeldung: **05.08.1998**
(21) Anmeldenummer: 98101225.5
(22) Anmeldetag: 24.01.1998
(51) Int. Cl.: A61B 5/14

(54) **Blutentnahmevorrichtung**

(30) Priorität: 29.01.1997 DE 19703178
(71) Anmelder: Sarstedt AG & Co., 51588 Nümbrecht (DE)
(72) Erfinder: Sarstedt, Walter, 51588 Nümbrecht (DE)
(74) Vertreter: Pollmeier, Felix, Dipl.-Ing.

(57) **Zusammenfassung**

Bei einer Blutentnahmevorrichtung, umfassend eine Röhrchenkappe (1) zum Verschließen eines Probennehmerröhrchens, die in einem Dom (2) eine scheibenförmige, durchstechbare, auf einer Auflagefläche aufliegende Membrane (5) aufweist, wird ein Schutz vor Kontaminationen erreicht, wenn der Dom-Hohlraum (3) zur tiefliegenden Anordnung der Membrane (5) genutzt ist, die in einer von der Dom-Oberkante (4) entfernten Position angeordnet ist.

## Beschreibung

Die Erfindung betrifft eine Blutentnahmevorrichtung, umfassend eine Röhrchenkappe zum Verschließen eines Probennehmerröhrchens, die in einem Dom eine scheibenförmige, durchstechbare, auf einer Auflagefläche aufliegende Membrane aufweist.

Eine Blutentnahmevorrichtung dieser Art ist durch die DE 29 48 653 C2 bekanntgeworden. Die elastische, zur Blutentnahme von beispielsweise einem Kanülenende einer Doppelkanüle durchstechbare Membrane ist dort unmittelbar am vorderen Ende des in diesem Fall exzentrischen Doms der Kappe angeordnet. Die scheibenförmige Membrane ist bei dieser bekannten Entnahmevorrichtung entweder durch eine starre Haltescheibe oder durch Umbördeln des oberen Ende der Domspitze - oder der Spitze eines zwischengeschalteten auf den Dom aufgesetzten Anpassungsstückes - gesichert bzw. in ihrer Lage festgelegt. Da sich beim Abziehen der Kanüle bzw. Doppelkanüle nach der Blutentnahme häufig nicht vermeiden läßt, daß Blut nach außen, d.h. auf die freie, außenliegende Oberfläche der Membrane gelangt, ist die Gefahr einer Kontamination nicht auszuschließen.

Um dieser Gefahr zu begegnen, sind Röhrchenkappen mit einem darin eingesetzten Stopfen bekannt, der mit einem innenliegenden, nach unten versetzten Boden ausgebildet ist, so daß eine die Blutentnahmevorrichtung handhabende Person nicht in Berührung mit dem sich in dieser Absenkung ansammelnden Blut gelangen bzw. mit dem Finger nicht darin eingreifen kann. Allerdings verkleinert der eingesetzte Stopfen den ohnehin nur sehr geringen Innendurchmesser des Hohlraums bzw. die Wandstärke des Doms wird durch die Wand des Stopfens vergrößert. Das kann dazu führen, daß das die angeschärfte Kanüle umschließende Ventilgummi beim Aufstecken der Kanüle auf den Dom und Durchstechen des abgesenkten Bodens eine nachteilige Reibschlußverbindung mit der Stopfenwandung eingeht, wobei anzumerken ist, daß sich das Ventilgummi beim Durchstechen des Stopfens ziehharmonikaartig zusammenschiebt und damit seinen Durchmesser vergrößert.

Schließlich sind Blutentnahme-Vorrichtungen bekannt (vgl. den Prospekt "VACUETTE, Das Vakuum-Blutentnahme-System aus Kunststoff", Greiner Labortechnik), bei denen ein wiederum einen abgesenkten Boden aufweisender Stopfen mit einer Kappe direkt auf das Probennehmerröhrchen aufgesteckt ist. Eine Röhrchenkappe mit einem Dom entfällt hierbei, da die Kanüle bzw. Doppelkanüle direkt auf den Mantel der den Stopfen aufnehmenden, das vordere Ende des Röhrchens umschließenden Kappe aufgesteckt wird. Trotz der hier - im Gegensatz zu einer Röhrchenkappe mit Dom und entsprechend kleiner Bohrung - sehr viel größeren Abmessungen wird durch die Absenkung im Stopfen dennoch ein Kontakt mit sich in der Absenkung ansammelndem Blut vermieden.

Der Erfindung liegt die Aufgabe zugrunde, bei einer gattungsgemäßen Blutentnahmevorrichtung eine Röhrchenkappe zu schaffen, die die genannten Nachteile nicht besitzt.

Diese Aufgabe wird erfindungsgemäß dadurch gelöst, daß der Dom-Hohlraum zur tiefliegenden Anordnung der Membrane genutzt ist, die in einer von der Dom-Oberkante entfernten Position angeordnet ist. Dieser Lösung liegt die Erkenntnis zugrunde, daß der Freiraum, der ansonsten in der Membrane bzw. dem Stopfen in Form einer Absenkung des durchzustoßenden Bodens liegt, erfindungsgemäß durch den ohnehin vorhandenen Hohlraum bzw. die Bohrung des Doms bereitgestellt werden kann. Trotz des geringen Bohrungsdurchmessers des Doms läßt sich auf diese Weise oberhalb der scheibenförmigen Membrane ein ausreichend großer Freiraum für sich ansammelndes Blut erreichen, ohne daß sich das ziehharmonikaartig zusammenschiebende Ventilgummi an die Domwandung anlegen kann, und außerdem ist es einer Bedienungsperson nicht möglich, mit einem Finger in diesen Hohlraum einzugreifen. Gleichzeitig ergibt sich ein sicherer Halt der in tiefliegender Lage in dem Dom eingesetzten scheibenförmigen Membrane, die nämlich von einem komplementären Formstempel mit größerem Druck als beispielsweise ein eingesetzter Stopfen mit der Domwandung verschweißt werden kann, denn schon im Bereich kurz oberhalb der Unterkante der scheibenförmigen Membrane ist eine diese ausreichend anschnäbelnde Einengung der Domwandung möglich. Nach dieser Einengung verläuft die Domwandung bis zur Dom-Oberkante entweder leicht konisch oder sogar zylindrisch aus, wobei sich in letzterem Fall ein größstmöglicher Durchmesser des von vornherein ohnehin schon geringen Dom-Durchmessers erreichen läßt.

Weitere Einzelheiten und Vorteile der Erfindung ergeben sich aus dem Patentanspruch und der nachfolgenden Beschreibung, in der - in stark vergrößertem Maßstab - Ausführungsbeispiele des Gegenstandes der Erfindung näher erläutert sind. Es zeigen:
- Fig. 1: eine Gesamtansicht einer ersten Ausführung einer Röhrchenkappe mit im wesentlichen konisch auslaufender Domspitze;
- Fig. 2: die Röhrchenkappe gemäß Fig. 1 im Teil-Längsschnitt dargestellt;
- Fig. 3: eine andere Ausführung einer Röhrchenkappe mit im wesentlichen einer zylindrisch auslaufenden Domspitze; und
- Fig. 4: die Röhrchenkappe gemäß Fig. 3 im Teil-Längsschnitt dargestellt.

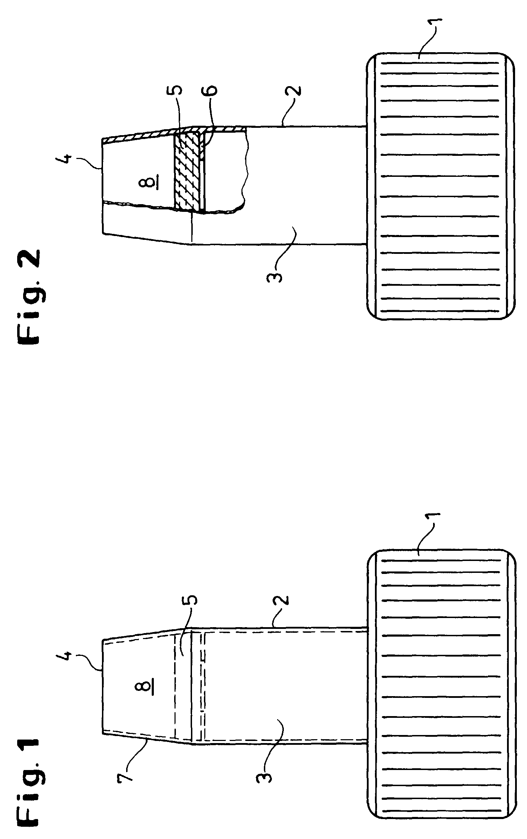

Die in Fig. 1 gezeigte, auf ein nicht dargestelltes Probennehmerröhrchen einer als solche hinlänglich bekannten Blutentnahmevorrichtung entweder aufschraubbare oder -steckbare Röhrchenkappe 1 besitzt einen Dom 2, dessen Hohlraum bzw, Innenbohrung 3 sogleich als Aufnahme für eine darin tiefliegend angeordnete, d.h. einen großen Abstand zu der Dom-Oberkante 4 einnehmende Membrane 5 genutzt bzw. verwendet ist. Die Membrane 5 liegt auf einer Auflagefläche 6 (vgl. Fig. 2) des Doms 2 auf und ist mit der Domwandung mittels eines nicht gezeigten Formstempels durch Aufbringen von äußerem Druck verschweißt worden. Der Druck läßt sich gezielt im Bereich des Außenmantels der Membrane 5 auf die Domwandung aufbringen, die dabei etwas oberhalb der Membran-Unterfläche in eine leichte Schräge mit ggf. einer leichten Rundung übergeht, wie näher der Fig. 2 zu entnehmen, danach bis zur Dom-Oberkante 4 schräg ausläuft und bei dieser Ausführung eine im wesentlichen konische Domspitze 7 bildet. Die Dom-Oberkante 4 ist nach innen leicht gerundet, womit einer möglichen Rißbildung vorgebeugt bzw. diese vermieden wird. Ohne Änderung des Doms 2, der nach wie vor einstückig ist, läßt sich durch die Anordnung der Membrane 6 im bzw. Ausnutzung der Bohrung bzw. des Hohlraums 3 des Doms 2 eine soweit untenliegende Lage der Membrane 5 erreichen, daß über ihrer Oberfläche ein solch großer Frei- bzw. Hohlraum 8 verbleibt, in den sich Blut ohne Verschleppungsgefahr ansammeln kann und der einer Bedienungsperson keine Eingriffsmöglichkeit bietet.

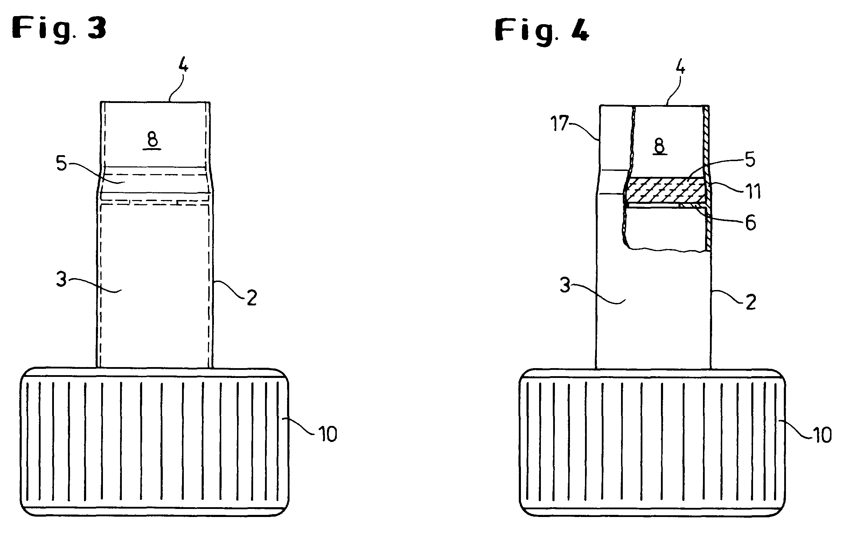

Die in den Fig. 3 und 4 gezeigte Röhrchenkappe 10 unterscheidet sich von der zuvor beschriebenen Röhrchenkappe 1 nur dadurch, daß die von dem Formstempel beim Verschweißen der Membrane 5 in eine im Anschluß an ine Prägschräge 11 bis zur Dom-Oberkante 4 im wesentlichen zylindrisch auslaufende Domspitze 17 übergeht.

Beiden Ausführungen, d.h. den Röhrchenkappen 1 und 10 ist gemeinsam, daß der vorhandene Dom-Hohlraum bzw. die Dom-Bohrung 3 zur tiefliegenden Anordnung der Membrane 5 genutzt ist, was trotz der geringen Dom- und damit Bohrungsdurchmesser oberhalb der Membrane 6 einen ausreichenden Hohlraum 8 schafft, so daß bei der Blutentnahme möglicherweise austretendes Blut sich darin ansammeln kann, ohne eine Kontaminations-Gefahr mit sich zu bringen.

## Patentansprüche

1. Blutentnahmevorrichtung, umfassend eine Röhrchenkappe zum Verschließen eines Probennehmerröhrchens, die in einem Dom eine scheibenförmige, durchstechbare, auf einer Auflagefläche aufliegende Membrane aufweist,
**dadurch gekennzeichnet,**
daß der Dom-Hohlraum (3) zur tiefliegenden Anordnung der Membrane (5) genutzt ist, die in einer von der Dom-Oberkante (4) entfernten Position angeordnet ist.
